# EUROPEAN PATENT APPLICATION

(11) **EP 4 438 045 A1**
(43) Date of publication of application: **02.10.2024**
(21) Application number: 22913154.5
(22) Date of filing: 02.06.2022
(51) Int. Cl.: A61K 31/53, A61P 1/16

(54) **USE OF ENHANCER 3C1 IN PREPARATION OF ENHANCER FOR INTERACTION OF FXR PROTEIN AND CASPASE 8 PROTEIN**

(30) Priority: 31.12.2021 CN 202111681403
(71) Applicant: China Pharmaceutical University, Nanjing, Jiangsu 211198 (CN)
(72) Inventor: HAO, Haiping, Nanjing, Jiangsu 211198 (CN); WANG, Hong, Nanjing, Jiangsu 211198 (CN); SUN, Huiyong, Nanjing, Jiangsu 211198 (CN); HU, Huijian, Nanjing, Jiangsu 211198 (CN); PAN, Xiaojie, Nanjing, Jiangsu 211198 (CN); WANG, Guangji, Nanjing, Jiangsu 211198 (CN); XU, Xiaowei, Nanjing, Jiangsu 211198 (CN); CUI, Shuang, Nanjing, Jiangsu 211198 (CN)
(74) Representative: Murgitroyd & Company
(86) International application number: PCT/CN2022/096810
(87) International publication number: WO 2023/123865

(57) **Abstract**

Provided in the present invention is the use of an enhancer named as 3C1 in the preparation of an enhancer for the interaction between FXR protein and Caspase 8 protein. The enhancer can promote the interaction between FXR protein and Caspase 8 protein, thereby inhibiting the recruitment of Caspase 8 to apoptosome and blocking its formation, inhibiting the cleavage and activation of Caspase 8, blocking signal transduction of apoptosis, and ultimately inhibiting cell apoptosis. Therefore, the enhancer can be used for preparing a drug for treating liver diseases characterized with hepatocyte apoptosis.

## Description

### TECHNICAL FIELD

The present invention relates to use of an enhancer in the preparation of an enhancer for protein-protein interaction, and particularly relates to use of an enhancer named as 3C1 in the preparation of an enhancer for the interaction between an FXR protein and a caspase 8 protein.

### BACKGROUND

Currently, the incidence of various acute and chronic liver diseases, including drug-induced liver injury, alcoholic fatty liver disease, nonalcoholic fatty liver disease, cholestatic liver injury, viral hepatitis, hepatic fibrosis, cirrhosis, liver cancer, and the like, remains high worldwide, seriously threatening human health. For example, about 185 million people worldwide are infected with viral hepatitis C. Besides, the prevalence of nonalcoholic fatty liver disease (NAFLD) exceeds 25%, with more than 100 million people progressing to nonalcoholic steatohepatitis (NASH). However, few specific drugs are currently available for the treatment of various hepatic diseases other than viral hepatitis C.

Cell apoptosis is a fundamental biological phenomenon that plays an important role in the evolution/development and homeostasis (internal environment) of organisms. However, disorders of cell apoptosis are closely related to the occurrence and development of a variety of diseases. For example, hepatocyte apoptosis is a common feature and pathological mechanism of various liver diseases, including drug-induced liver injury, alcoholic liver injury, NALFD/NASH, viral hepatitis, cholestatic liver injury, hepatic ischemia reperfusion injury, hepatolenticular degeneration, hepatic fibrosis/cirrhosis, liver cancer, and the like. Inhibiting hepatocyte apoptosis is widely accepted as one of the important strategies for the treatment of liver diseases (Cellular and Molecular Mechanisms of liver Injury. Gastroenterology, 2008, 134(6): 1641-54.).

As a member of a nuclear receptor superfamily, Farnesoid X Receptor (FXR, also known as NR1H4) plays an important role in bile acid, glucose, and lipid metabolism, and is also closely related to physiological and pathological processes, such as energy balance, inflammation, cell fate, fibrosis, and liver regeneration. Therefore, the FXR is widely deemed as an important target for the development of hepatoprotective drugs (FXR Modulators for Enterohepatic and Metabolic Diseases. Expert Opin Ther Pat. 2018 Nov; 28(11):765-782.). Currently, strategies of drug development targeting FXR mainly focus on the development of FXR agonists or antagonists to regulate its transcriptional activity. Obeticholic acid, an FXR agonist, was approved by the US FDA for the treatment of primary biliary cirrhosis in May 2016. It is the first drug that successfully marketed with FXR as the target. A phase III Clinical trial to evaluate the effect of the obeticholic acid on NASH have been completed. However, results showed that its clinical efficacy is far from satisfactory (Obeticholic Acid for the Treatment of Non-alcoholic Steatohepatitis: Interim Analysis from a Multicentre, Randomised, Placebo-controlled Phase 3 Trial. Lancet. 2019 Dec 14; 394(10215):2184-2196).

Studies indicate that the NASH and hepatic fibrosis are accompanied by excessive hepatocyte apoptosis. FXR agonists failed to alleviate hepatocyte apoptosis in states of the NASH and other diseases, which is one of the main reasons for its limited clinical efficacy (Combined Obeticholic Acid and Apoptosis Inhibitor Treatment Alleviates Hepatic Fibrosis. Acta Pharm Sin B. 2019 May; 9(3):526-536.). Further, previous studies by the research group have shown a stable interaction between an FXR protein and a Caspase 8 protein in hepatocytes. The interaction between FXR protein and Caspase 8 protein is weakened in apoptotic hepatocytes, facilitating the association of Caspase 8 protein to FAS-Associated Death Domain (FADD) and receptor-interacting protein 1 (RIP1) to form apoptosome, and subsequent cleavage and activation of the Caspase 8 protein and the progression of apoptosis (Noncanonical Farnesoid X Receptor Signaling Inhibits Apoptosis and Impedes Hepatic Fibrosis. EBioMedicine. 2018 Nov; 37:322-333.). Therefore, stabilizing or enhancing the interaction between FXR protein and Caspase 8 protein is an important strategy to prevent apoptosis. However, it remains unknown about the regulation of interaction between the FXR protein and the Caspase 8 protein, and there is currently no regulator of interaction between the FXR protein and the Caspase 8 protein.

### SUMMARY

One objective of the present invention is to provide use of an enhancer named as 3C1 in the preparation of an enhancer for the interaction between FXR protein and Caspase 8 protein. Another objective is to provide the use of this enhancer named as 3C1 in preparing drugs for the treatment of hepatic fibrosis and liver injury, characterized as hepatocellular apoptosis.

Technical solution: the present invention provides the use of an enhancer named as 3C1 in the preparation of an enhancer for the interaction between FXR protein and Caspase 8 protein in Formula (1) of the present invention:

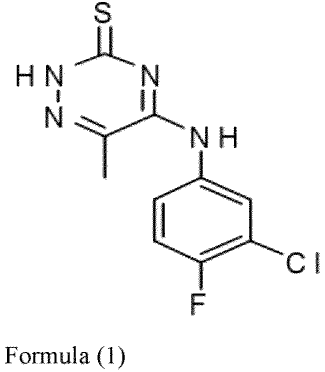

The enhancer 3C1 promotes the interaction between FXR protein and Caspase 8 protein under physiological or pathological conditions.

The enhancer prevents formation of an apoptosome by blocking the recruitment of Caspase 8 protein to the apoptosome.

The enhancer inhibits cleavage and activation of Caspase 8 protein.

Pharmaceutically acceptable excipients are added for the enhancer to prepare a preparation.

The pharmaceutically acceptable excipients include a diluent, a binder, a disintegrant, a glidant, a lubricant, a corrigent, an inclusion material, and an adsorbent material.

The preparation exists in the form of granules, powders, tablets, capsules, pills, or oral liquids.

The use of an enhancer named as 3C1 as an enhancer for the interaction between FXR protein and Caspase 8 protein in preparing drugs for combating hepatocyte apoptosis is disclosed.

The use of an enhancer named as 3C1 as an enhancer for the interaction between FXR protein and Caspase 8 protein in preparing hepatoprotective drugs is disclosed.

The use of an enhancer named as 3C1 as an enhancer for the interaction between FXR protein and Caspase 8 protein in preparing drugs for the treatment of hepatic fibrosis and liver injury is disclosed.

Beneficial effects: compared with the prior art, the present invention has the following significant advantages: (1) compared to the control group incubated with vehicle, the enhancer can significantly enhance the interaction between FXR protein and Caspase 8 protein in physiological state, with an optimal value reaching ***P<0.001; (2) the enhancer can restore and enhance the weakened interaction between FXR protein and Caspase 8 protein in apoptotic hepatocytes; (3) compared to the control group incubated with vehicle, the enhancer can significantly combat hepatocyte apoptosis caused by various stimulation, with an optimum value reaching ***P<0.001; and (4) the enhancer has a precise drug target, high specificity, strong specificity, and a wide range of indications, and can be used for preparing drugs for the treatment of various liver diseases characterized with apoptotic hepatocytes.

### BRIEF DESCRIPTION OF THE DRAWINGS

FIG. 1 shows an interaction between FXR protein and Caspase 8 protein in apoptotic hepatocytes.
FIG. 2 shows the chemical structural formula of the enhancer 3C1 of the present invention.
FIG. 3 shows the effect of the enhancer 3C1 of the present invention in regulating the interaction between FXR protein and Caspase 8 protein in healthy cells.
FIG. 4 shows the effect of the enhancer 3C1 of the present invention in regulating the interaction between FXR protein and Caspase 8 protein in apoptotic cells.
FIG. 5 shows the effect of the enhancer 3C1 of the present invention in preventing apoptosis, (A) hepatocyte apoptosis induced by Actinomycin D (ActD)/tumor necrosis factor alpha (TNFα); (B) hepatocyte apoptosis induced by cycloheximide (CHX)/Fas ligand (FasL); and (C) hepatocyte apoptosis induced by a human tumor necrosis factor-related apoptosis-inducing ligand (TRAIL), where *P<0.05, **P<0.01, and ***P<0.001.
FIG. 6 shows the effect of enhancer 3C1 in alleviating carbon tetrachloride (CCl₄)-induced liver injury, (A) serum levels of alanine transaminase (ALT); (B) serum levels of aspartate transaminase (AST); (C) hematoxylin and eosin (H&E) staining of liver tissues; (D) Sirius red staining of liver tissues; (E) mRNA levels of tissue inhibitor of metalloproteinases-1 (Timp1) in liver; and (F) mRNAlevels of Timp2 in liver, where *P<0.05, **P<0.01, and ***P<0.001.

### DETAILED DESCRIPTIONS OF THE EMBODIMENTS

### Example 1

Changes about the interaction between Farnesoid X Receptor (FXR) protein and Caspase 8 protein in apoptotic hepatocytes.

### 1. Experimental materials

Dulbecco's modified eagle medium (DMEM) was purchased from GIBCO (Grand Island, New York, USA). Fetal bovine serum (FBS) was purchased from Hyclone (Logan, Utah, USA). Trypsin was purchased from Amersco (Solon, Ohio, USA). Cell plates was purchased from Costar (USA). Actinomycin D (ActD) and cycloheximide (CHX) were purchased from MedChem Express (NJ, USA). Recombinant human tumor necrosis factor alpha (TNFα) was purchased from Peprotech (Rocky Hill, USA), and recombinant human FasL protein and recombinant human TRAIL protein were purchased from Sino Biological (Wayne, PA, USA).

### 2. Experimental methods

### 2.1 Cell culture and administration

A human hepatocyte cell line L02 was cultured in a complete DMEM containing 10% FBS, and placed in a culture incubator containing 5% CO₂ for culturing at 37°C, and cells in a logarithmic growth phase were inoculated in a 12-well plate at a density of 2×10⁵ cells/well. After 24 h, an apoptosis model was constructed by means of human tumor necrosis factor-related apoptosis-inducing ligand (TRAIL), Actinomycin D (ActD)/tumor necrosis factor alpha (TNFα), and cycloheximide (CHX)/Fas ligand (FasL) methods respectively. Cells were treated with TRAIL at 50 ng/ml for 0, 2, 4, 8, 12, and 24 h, respectively. Apoptosis was also induced by incubation with ActD (0.2 µM) for 30 min followed by the addition of TNFα (20 ng/ml). Cells were incubated with ActD /TNFα for 0, 1, 2, 4, 8, and 12 h, respectively. Apoptosis was also induced by incubation with CHX (50 µM) for 30 min followed by the addition of FasL (50 ng/ml). Cells were incubated with ActD /TNFα for 0, 2, 4, and 8 h, respectively.

### 2.2 Co-immunoprecipitation (Co-IP)

1) Cells were washed twice with pre-chilled phosphate-buffered saline (PBS), an appropriate amount of pre-chilled PBS was added for the last time, a pre-chilled cell scraper was used to scrape the cells from a culture dish or a culture flask, and a suspension was transferred to a 1.5 ml electropolished (EP) tube. After centrifugation at 4°C and 4000 rpm for 5 min, a supernatant was discarded, an appropriate amount of an NP40 lysis buffer was added, a mixture obtained was shaken evenly, and the cells were ultrasonicated for a total of 3 times (5 s per time) until the cells were completely lysed. After centrifugation at 4°C and 14000 g for 10 min, a supernatant was transferred to a new EP tube, to obtain a protein lysis buffer. A protein concentration was determined through a bicinchoninic acid (BCA) assay.
2) 25 µl of Protein A Beads was taken and blocked with 5% bovine serum albumin (BSA) for 1 h at room temperature, and then washed with the PBS for 3 times, and a supernatant was discarded after centrifugation at 14000 g for 1 min each time. The protein lysis buffer with a uniform concentration was added to the Beads, rocking-turn was performed at 4°C for 1 h, and a sample was pre-cleared. After centrifugation at 14000 g for 1 min, a supernatant was transferred to a new EP tube. A Caspase 8 protein antibody and an IgG antibody were added to the protein lysis buffer, and shaking at 4°C was performed overnight to prepare an antigen-antibody complex. After the Beads were washed twice next day, centrifugation was performed at 14000 g and 4°C for 1 min, a mixed solution of antigen-antibody (Ag-Ab) was added to the Beads, and a mixture obtained was shaken at 4°C for 3 h to prepare a Beads-antigen-antibody complex. Centrifugation was then performed at 14000 g and 4°C for 1 min. A supernatant was discarded and the bottom (Beads) thereof was left. After the Beads were washed for 3 times, instantaneous centrifugation was performed. A 2× Loading Buffer (30 µl) was added to the Beads, and a mixture obtained was cooked in boiling water for 5 min, and then centrifuged at 12000 rpm for 5 min. Western blot and gel imaging analysis were performed.

### 2.3 Western blot

1) The cells were collected and placed on ice, 100 µl of a Radio-Immunoprecipitation Assay (RIPA) lysis buffer, a 1% Protease Inhibitor Cocktail (Sigma), and a 1% phosphatase inhibitor was added to each 20 µl of packed cell volume, clumps were fully dispersed, and after lysis on ice for 30 min, the cells were ultrasonicated for 5 times (2 s per time), where pausing was performed for a few seconds to allow the lysis buffer to cool in the process. Centrifugation was performed at 13000 g for 10 min to obtain a supernatant as an extracted total protein sample, the sample was transferred to a new centrifuge tube, a protein content thereof was determined based on the BCA method, a 4× loading buffer was added, and boiling for 10 min after mixing well was performed for sample loading.
2) Sodium dodecyl sulfate-polyacrylamide gel electrophoresis (SDS-PAGE) containing 3%-5% spacer gel and 6%-12% separation gel was prepared, and a protein sample was separated by electrophoresis. The sample was shaken well, and the same amount (50-100 µg) of total protein per sample was taken for sample loading. Electrophoresis in the spacer gel was performed at a constant voltage of 75 V for 40 min, and gradient electrophoresis in the separation gel was performed at a constant voltage of 115 V for 60 min. Protein transfer was performed under semi-dry transfer conditions, and filter paper, cut gel and a polyvinylidene fluoride (PVDF) membrane were equilibrated in a semi-dry transfer buffer, where the filter paper was placed flatwise in a semi-dry transfer tank, the PVDF membrane, the gel and another piece of filter paper were sequentially placed on the top, and all bubbles between layers were carefully removed. A semi-dry transfer lid was buckled on the tank, the constant voltage was set to 25V, and the transfer was performed for 30 min.
3) After the transfer, the PVDF membrane was blocked in a 5% skim milk blocking solution prepared in a Tris Buffered Saline with Tween^{®} 20 (TBST) solution, and blocking was performed at 37°C for 1 h. An appropriate amount of primary antibody prepared in the blocking solution was added according to a relative amount (µl/cm²) of the PVDF membrane, and incubating was performed at 4°C overnight. The PVDF membrane was rinsed with the TBST solution for 4 times (5 min per time). The PVDF membrane was incubated at 37°C in a horseradish peroxidase (HRP)-conjugated secondary antibody dilution buffer for 60 min, and the PVDF membrane was rinsed in the TBST solution for 4 times (5 min per time).
4) Exposure was performed through a ChemiDoc^{™} XRS and a gel imaging system (Bio-Rad), and Image Lab TM software was used for densitometry analysis and semi-quantitative comparison.

### 3. Experimental results

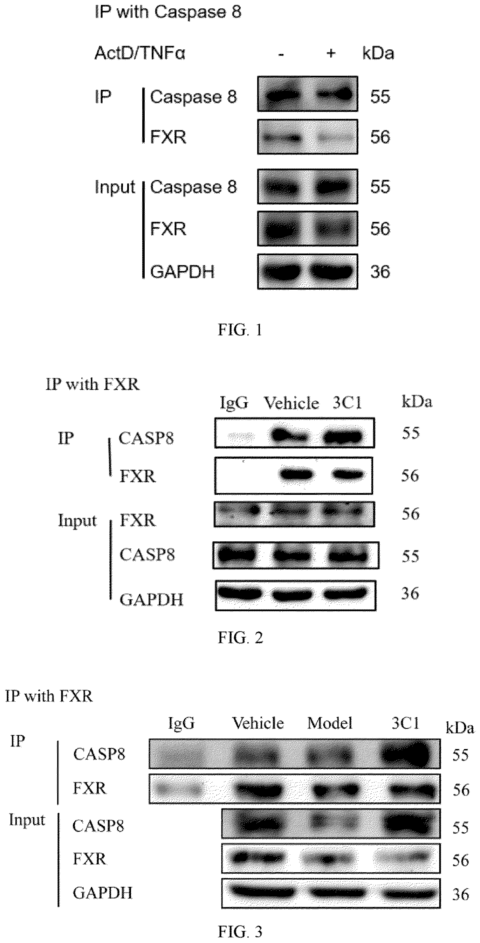

As shown in FIG. 1, , the FXR protein interacted with the Caspase 8 protein under physiological conditions, while in the ActD/TNFα-induced apoptosis model, the interaction between the FXR protein and the Caspase 8 protein was weakened.

### Example 2

Effect of the enhancer 3C1 in regulating the interaction between Farnesoid X Receptor (FXR) protein and Caspase 8 protein

### 1. Experimental materials

Compound 3C1 was purchased from Topscience, and its structural formula is as follows:

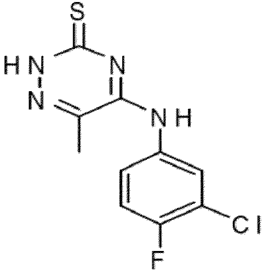

All other reagents are the same as those in Example 1.

### 2. Experimental methods

### 2.1 Cell culture and administration

Human hepatoyte cell line L02 was cultured in complete dulbecco's modified eagle medium (DMEM) containing 10% FBS, and placed in a culture incubator containing 5% CO₂ for culturing at 37°C.

### 2.2 Effect of the compound 3C1 in regulating the interaction between FXR protein and Caspase 8 protein under physiological conditions

Cells in a logarithmic growth phase were inoculated in a 6-well plate at a density of 2×10⁵ cells/well. After 24 h, the compound 3C1 of different concentrations (1 µM, 10 µM, and 100 µM) was administered, and cells were collected after 2 h.

### 2.3 Effect of the compound 3C1 in regulating the interaction between FXR protein and Caspase 8 protein under apoptotic conditions

Cells in a logarithmic growth phase were inoculated in a 6-well plate at a density of 2×10⁵ cells/well. Apoptosis was induced as described in Example 1. In addition to an apoptotic inducer, the compound 3C1 of different concentrations (1 µM, 10 µM, and 100 µM) was administered, and cells were collected after 2 h.

### 2.4 Co-immunoprecipitation (Co-IP)

It is the same as that in Example 1.

### 2.5 Western blot

It is the same as that in Example 1.

### 3. Experimental results

### 3.1 Effect of compound 3C1 in regulating the interaction between the FXR protein and the Caspase 8 protein in healthy hepatocytes

As shown in FIG. 2, treatment with compound 3C1 obviously enhanced the interaction between FXR protein and Caspase 8 protein in healthy cells in a dose-dependent manner.

### 3.2 Effect of compound 3C1 in regulating the interaction between FXR protein and Caspase 8 protein in apoptotic hepatocytes

As described in Example 1, the interaction between FXR protein and Caspase 8 protein was reduced in apoptotic hepatocytes. As shown in FIG. 3, the interaction between FXR protein and Caspase 8 protein was weakened apoptotic hepatocytes caused by Actinomycin D (ActD)/tumor necrosis factor alpha (TNFα) treatment, while incubation with compound 3C1 could reverse this reduction.

The above results prove that compound 3C1 is an enhancer for the interaction between FXR protein and Caspase 8 protein.

### Example 3

Pharmacological activity of the enhancer 3C1 against apoptosis

### 1. Experimental materials

Methylthiazolyldiphenyl-tetrazolium bromide (MTT) was purchased from MedChem Express.

All other reagents are the same as those in Example 1.

### 2. Experimental methods

### 2.1 Cell culture, transfection and administration

A human hepatocyte cell line L02 was cultured in complete medium DMEM containing 10% fetal bovine serum (FBS), and placed in a culture incubator containing 5% CO₂ at 37°C, and cells in a logarithmic growth phase were inoculated in a 96-well plate at a density of 5×10³ cells/well. The cells were transfected at 50 nM after 24 h. Hepatocyte apoptosis was induced 48 h later after the transfection. Apoptosis was induced by TRAIL treatment at 50 ng/ml for 24 h. Apoptosis was also induced by incubation with ActD (0.2 µM) for 30 min followed by the addition of TNFα (20 ng/ml) for 8 h. Apoptosis was also induced by incubation with CHX (50 µM) for 30 min followed by the addition of FasL (50 ng/ml) for 8 h.

In addition to an apoptosis reagent, the compound 3C1 of different concentrations (1 µM, 10 µM, and 100 µM) was administered.

### 2.2 Cell viability assay

After the time of administration, 20 µl of MTT (5 mg/ml) was added to each well, the cells were further incubated at 37°C for 4 h, a culture medium was aspirated and discarded, 150 µl of dimethyl sulfoxide (DMSO) was added to each well, incubation was performed at 37°C for 10 min, and the absorbance was measured at 490 nm.

### 3. Experimental results

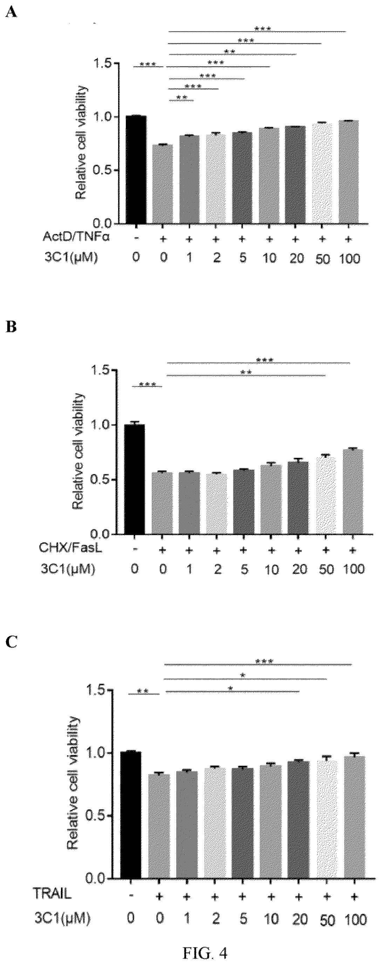

As shown in FIG. 4, the enhancer 3C1 of FXR and Caspase 8 protein complex showed significant anti-apoptotic effects in three classical apoptosis models of TRAIL (FIG. 4A), ActD/TNFα (FIG. 4B), and CHX/FasL (FIG. 4C) in a dose-dependent manner.

### Example 4

Effect of the enhancer 3C1 in alleviating carbon tetrachloride (CCl₄)-induced liver injury

### 1. Experimental materials

Experimental mice (C57BL/6) were purchased from Beijing Vital River Laboratory Animal Technology Co., Ltd.

CCl₄ was purchased from Shanghai Lingfeng Chemical Reagent Co., Ltd., mineral oil was purchased from Sigma-Aldrich, Trizol RNAiso plus was purchased from Takara, reverse transcription kits and SYBR Green were purchased from Applied Biosystems, and the primers used were designed and synthesized by Invitrogen (Shanghai, China). Diethyl pyrocarbonate (DEPC) water was purchased from Beyotime Institute of Biotechnology (Shanghai, China).

All other biological materials/reagents can be obtained from commercial sources.

All other experimental materials are the same as those in Example 1.

### 2. Experimental methods

### 2.1 Effect of the enhancer 3C1 in preventing CCl₄-induced liver injury of mice

Before the experiment, C57BL/6 mice were acclimated for one week under the conditions of, room temperature at 22-26°C and relative humidity of 40-60%, with light alternated with darkness for 12 h. Each mouse was housed in a separate cage with free access to water and food, and their body weights were maintained at 20-22 g. Experimental groups included a blank control group, a model group, a low-dose 3C1 administration group (5 mg/kg), and a high-dose 3C1 administration group (10 mg/kg).

The C57BL/6 mice in the low-dose 3C1 administration group and the high-dose 3C1 administration group were pre-administered with intraperitoneal injections of 3C1 at a dose of 5 mg/kg/day or 10 mg/kg/day, and the mice in other groups were subject to vehicle control. 3 days later, liver injury was induced by intraperitoneal injections of CCl₄, and the control group was administered with mineral oil. The mice were euthanized 48 h later. Blood and livers of the mice were collected.

### 2.2 Serum transaminase assay

After standing at room temperature for 0.5 h, whole blood was centrifuged at 8000 rpm for 10 min, and a supernatant was transferred to a new electropolished (EP) tube to obtain serum. Alanine transaminase (ALT) and aspartate transaminase (AST) were detected according to instructions of ALT and AST assay kits.

### 2.3 Reverse Transcription-Polymerase Chain Reaction (RT-PCR)

### 2.3.1 Total ribonucleic acid (RNA) extraction from animal tissue samples

1) 20 mg of a liver tissue sample from each mouse was weighed, 1.0 ml of RNAiso Plus was added, the samples were homogenized, and a homogenate was transferred to a centrifuge tubes, kept standing at room temperature for 5 min, and centrifuged at 4°C and 12000g for 5 min.
2) 800 µl of a supernatant was transferred, 160 µl of chloroform was added, and a mixture obtained was vigorously shaken for 15 sec, and then centrifuged at 12000g for 15 min after standing at room temperature for 5 min. A sample included three layers: a bottom layer as a yellow organic phase, an upper layer as a colorless aqueous phase, and an intermediate layer.
3) 300 µl of the upper aqueous phase was carefully transferred to a new tube, 300 µl of isopropanol was added, after inverting and mixing, a mixture obtained stood at room temperature for 10 min, and after centrifugation at 12000 g for 10 min, the supernatant was discarded.

An RNA precipitate was washed with 1.0 ml of pre-chilled 75% ethanol and then centrifuged at 12000g for 5 min, and a supernatant was discarded to obtain total RNA, which was reconstituted with 20 µl of DEPC water and diluted to 0.5 µg/µl after quantification.

### 2.3.2 Reverse transcription

According to a system ratio indicated in the specification, the RNA solution and kit components were prepared into a system with a total volume of 20 µl, and a programmed temperature was set for reverse transcription. Specific ratio requirements were as follows:

| Reagent | Dosage |
|---|---|
| 10*RT Buffer | 2.0 µl |
| 25*dNTP Mix (100mM) | 0.8 µl |
| 10*RT Random Primers | 2.0 µl |
| MultiScribeReverseTransscriptase | 1.0 µl |
| Rnase free dH2O | 4.2 µl |
| Total | 10 µl |
| Total RNA | 10 µl |

### 2.3.3 PCR

The PCR system is as follows:

| Reagent | Dosage |
|---|---|
| SYBR Green | 7.5 µl |
| PCR Forward Primer (10 µM) | 1.0 µl |
| PCR Reverse Primer (10 µM) | 1.0 µl |
| cDNA | 1.0 µl |
| Rnase free dH2O | 4.5 µl |
| Total | 15.0 µl |

PCR use conditions are as follows:
Stage 1 : Pre-denaturation at 95°C for 1 min
Stage2: PCR reaction at 95°C for 15 sec; at 60°C for 30 sec for 40 cycles; and at 72°C for 30 sec
Stage 3: Melting curve analysis, 65-95°C, 0.5°C/5 sec

### 2.3.4 The primers used for quantitative fluorescence PCR are shown in Table 1:

**Table 1 Primers used for quantitative fluorescence PCR**

| Gene | Sequence |
|---|---|
| Timp1 | CATGGAAAGCCTCTGTGGATATG GATGTGCAAATTTCCGTTCCTT |
| Timp2 | ATGGTTCTTGCGCGTGGTA GCTTTTCAATTGGCCACAGG |
| Gapdh | TTGATGGCAACAATCTCCAC CGTCCCGTAGACAAAATGGT |

### 2.4 Liver pathology analysis

Wuhan Servicebio Co., Ltd. was entrusted to complete H&E staining and Sirius Red staining.

### 3. Experimental results

### 3.1 Effect of the enhancer 3C1 on serum biochemical indices

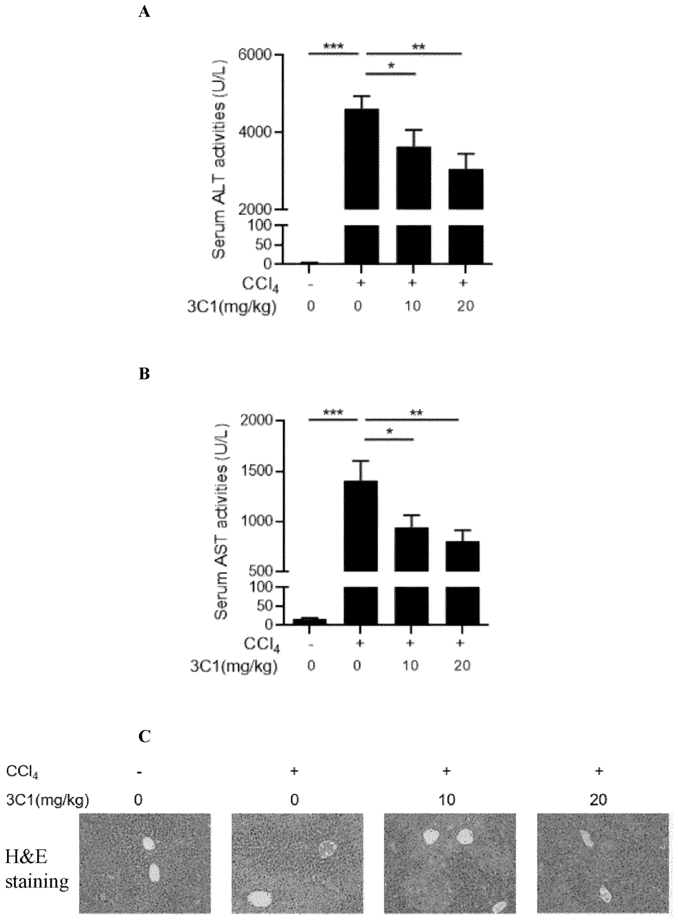

As shown in FIGs. 5A and 5B, CCl₄ injection significantly increased the levels of serum ALT and AST in mice, while the enhancer 3C1 significantly reduced the elevated levels of serum ALT and AST caused by CCl₄ injection.

### 3.2 Improvement of liver pathology by the enhancer 3C1

According to the liver pathology analysis of H&E staining results (FIG. 5C), liver lobule structures of the mice in the control group were intact, and hepatocytes were arranged radially around the central vein, without fibrosis or inflammatory cells. The hepatocytes were large, with round nuclei located centrally. After CCl₄ injection, hepatic sinusoids were significantly congested and dilated, the hepatocytes showed granular changes, some hepatocytes were infected with massive necrotic foci, some hepatocytes exhibited edema and degeneration, infiltration of inflammatory cell and disruption of some liver lobule structures were observed, fibrous tissues in a portal area showed dysplasia, and there was obvious band-like collagen fiber hyperplasia. The mice in the 3C1 administration group showed significant remission in the disorders of liver lobule structures, decreased infiltration of inflammatory cell, and less collagen fibers in the portal area. This indicates that the enhancer 3C1 had a significant hepatoprotective effect.

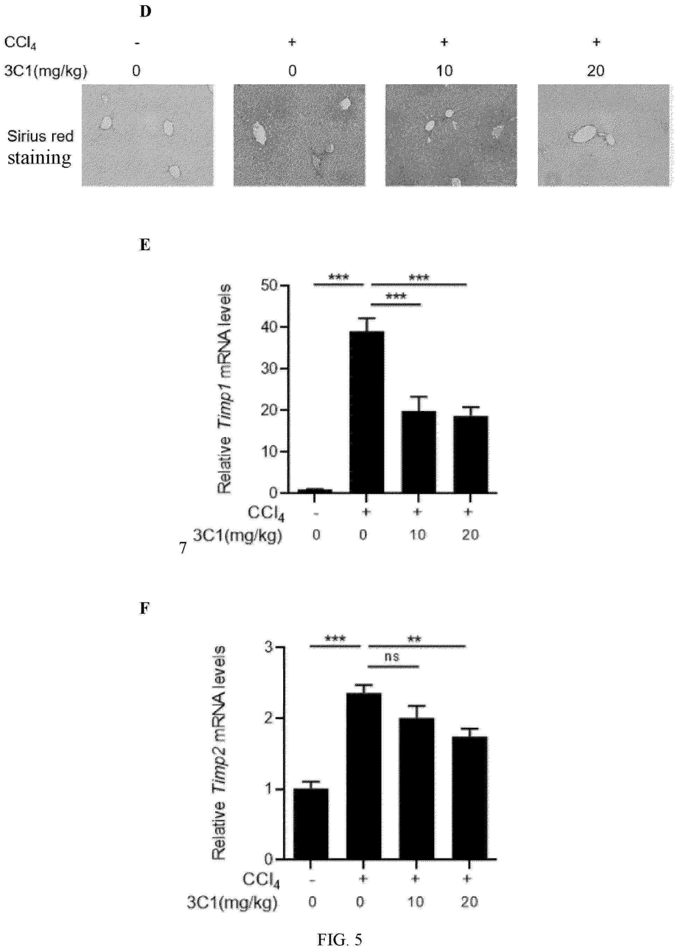

According to the liver pathology analysis of Sirius Red staining results (FIG. 5D), only less red collagen fibers in the livers of mice in the control group were stained, after the CCl₄ injection, a large number of red collagen fibers were observed in the portal area, and after administration with 3C1, hyperplasia of red collagen fibers in the livers was significantly alleviated.

### 3.3 Improvement of hepatic fibrosis by the enhancer 3C1

According to the results of real-time RT-PCR (FIGs. 5E and 5F), in a CCl₄-induced acute liver injury model for mice, mRNA levels of tissue inhibitor of metalloproteinases-1 (Timp1) and Timp2 were significantly increased (P < 0.001), and the enhancer 3C1 significantly reduced their mRNA levels (P < 0.001) in a dose-dependent manner.

To sum up, the enhancer 3C1 has a definite hepatoprotective effect.

### Example 5

50 mg of an enhancer 3C1 was mixed with 50 mg of microcrystalline cellulose and 60 mg of starch, an appropriate amount of 20% ethanol was used as a wetting agent to prepare a soft material, which was then granulated by means of a conventional method, talcum powder and magnesium stearate were added and mixed, and a mixture obtained was compressed into tablets.

50 mg of the enhancer 3C1 was mixed with 50 mg of starch, 10 mg of dextrin, and 10 mg of sucrose, and 30% ethanol was used as a wetting agent to prepare a soft material, which was then granulated by means of a wet method.

50 mg of the enhancer 3C1 was mixed with 50 mg of starch, 10 mg of dextrin, and 10 mg of sucrose, and 30% ethanol was used as a wetting agent to prepare a soft material, which was then granulated by means of a wet method and finally capsulated to produce capsules.

50 mg of the enhancer 3C1 was mixed with 40 mg of starch and 10 mg of talcum powder to produce a powder formulation by means of conventional techniques.

50 mg of the enhancer 3C1 was mixed with 15 mg of powdered sugar and 8 mL of water to produce a syrup formulation by means of conventional techniques, and then 70 mg of starch was added to produce a pill formulation through conventional techniques.

50 mg of the enhancer 3C1 was mixed with povidone, sucrose and water to produce an oral solution through conventional techniques.

## Claims

1. Use of an enhancer named as 3C1 in the preparation of an enhancer for the interaction between FXR protein and Caspase 8 protein as shown in Formula (1):

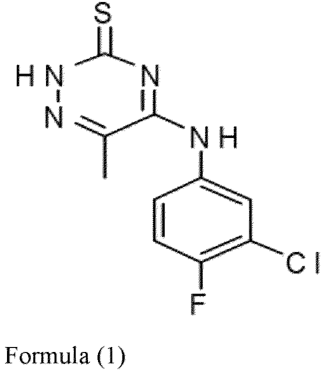

2. The use according to claim 1, wherein the enhancer 3C1 promotes the interaction between FXR protein and Caspase 8 protein under physiological or pathological conditions.

3. The use according to claim 1, wherein the enhancer prevents formation of apoptosome by blocking recruitment of Caspase 8 protein to the apoptosome.

4. The use according to claim 1, wherein the enhancer inhibits cleavage and activation of Caspase 8 protein.

5. The use according to claim 1, wherein pharmaceutically acceptable excipients are added for the enhancer to prepare a preparation.

6. The use according to claim 5, wherein the pharmaceutically acceptable excipients comprise a diluent, a binder, a disintegrant, a glidant, a lubricant, a corrigent, an inclusion material, and an adsorbent material.

7. The use according to claim 5, wherein the preparation exists in the form of granules, powders, tablets, capsules, pills, or oral liquids.

8. The use of an enhancer named as 3C1 as an enhancer for the interaction between FXR protein and Caspase 8 protein in preparing drugs for combating hepatocyte apoptosis.

9. The use of an enhancer named as 3C1 as enhancer for the interaction between FXR protein and Caspase 8 protein in preparing hepatoprotective drugs.

10. The use of an enhancer named as 3C1 as an enhancer for the interaction between FXR protein and Caspase 8 protein in preparing drugs for the treatment of hepatic fibrosis and liver injury.
